# EUROPÄISCHE PATENTANMELDUNG

(11) **EP 4 437 947 A1**
(43) Veröffentlichungstag der Anmeldung: **02.10.2024**
(21) Anmeldenummer: 23166016.8
(22) Anmeldetag: 31.03.2023
(51) Int. Cl.: A61B 5/0536

(54) **VORRICHTUNG UND VERFAHREN ZUR NICHT INVASIVEN BESTIMMUNG EINES VERSCHLUSSDRUCKS IN DER LUNGE EINES PATIENTEN**

(71) Anmelder: SenTec AG, 4106 Therwil (CH)
(72) Erfinder: Böhm, Stephan Hubertus, 4930 Maribo (DK); Pulletz, Sven, 49090 Osnabrück (DE)
(74) Vertreter: Hepp Wenger Ryffel AG

(57) **Zusammenfassung**

Die Erfindung betrifft eine Vorrichtung und ein Verfahren zur nicht invasiven Bestimmung einer Grösse, die repräsentativ für den Verschlussdruck ist, sowie ein System mit einem Beatmungsgerät, ein Computerprogramm und ein Computerprogrammprodukt.

Die Vorrichtung (10) umfasst einen Eingang (13) für Daten eines Beatmungsdrucks von einem Beatmungsgerät (30) über mindestens einen Beatmungszyklus, einen Eingang (12) für EIT-Daten von einem EIT-Gerät (20), die während des mindestens eines Beatmungszyklus erhoben wurden, und eine Recheneinheit (14). Diese ist dazu ausgelegt, auf Grundlage der Daten des Beatmungsgeräts und des EIT-Geräts die erste zeitliche Ableitung von EIT-Werten in Abhängigkeit des Beatmungsdrucks über mindestens einen Beatmungszyklus zu bestimmen.

Die Recheneinheit (14) ist weiter dazu ausgelegt, zumindest eine charakteristische Eigenschaft der Kurve, die repräsentativ für den Verschlussdruck ist, insbesondere einen charakteristischen exspiratorischen Kurvenpunkt, weiter insbesondere den Deflektionspunkt, den Wendepunkt und/oder das Minimum, der ersten zeitliche Ableitung der EIT-Werte in Abhängigkeit des Beatmungsdrucks zu bestimmen.

## Beschreibung

Die Erfindung betrifft eine Vorrichtung und ein Verfahren zur nicht invasiven Bestimmung einer Grösse, die repräsentativ für einen Verschlussdruck in der Lunge eines Patienten ist, sowie ein System mit einem Beatmungsgerät, ein Computerprogramm und ein Computerprogrammprodukt.

Jährlich werden mehr als 230 Millionen Menschen intraoperativ maschinell beatmet, um die respiratorische Funktion der Lunge zu unterstützen. Zusätzlich wird eine grosse Zahl von Patienten, teilweise über eine längere Zeit, auf Intensivstationen beatmet.

Die maschinelle Beatmung wird kontrovers angesehen, da sie Risiken und Komplikationen kardiovaskulärer und pulmonaler Art mit sich bringen kann, obwohl sie die Protektion bzw. Therapie jenes Organsystems zum Ziel haben sollte. Bei jeder Beatmungsindikation besteht die Möglichkeit, auch bei einer zuvor gesunden Lunge, einen Lungenschaden durch maschinelle Beatmung auszulösen. Als schädigende Grössen haben sich insbesondere hohe Beatmungsdrücke herausgestellt, die einen erhöhten transpulmonalen Druck (TPP) bis hin zum Barotrauma mit sich bringen.

Die Wahl einer geeigneten Beatmungsform sowie die Einstellung der Beatmungsparameter für das Erreichen von hinreichender Ventilation und Oxygenierung, ohne die Lunge zu schädigen, stellen demnach stets eine Herausforderung dar.

Der transpulmonale Druck ist die Druckdifferenz zwischen dem intrapulmonalem und dem extrapulmonalen Druck, anders ausgedrückt zwischen dem Alveolardruck und dem Pleuradruck. Der transpulmonale Druck bestimmt massgeblich das verfügbare Lumen der peripheren Lungenanteile (Bronchiolen, Lungenalveolen).

Der transpulmonale Druck bestimmt die Dehnung und das Volumen der Alveolen. Die respiratorische Änderung des transpulmonalen Drucks bewirkt Ein- und Ausstrom der Atemluft.

Ein Alveolarkollaps kann bei negativem transpulmonalem Druck eintreten und eine alveoläre Überdehnung kann bei einem zu hohen transpulmonalen Druck eintreten, z. B. bei einer invasiven Beatmung.

Eine Überdehnung oder ein Kollabieren von Alveolen kann verhindert werden und gleichzeitig möglichst wenig Spannung in der einzelnen Alveole erzeugt werden, indem man in Abhängigkeit der Beatmungseinstellungen transpulmonale Druckwerte von kleiner als 25 mbar anstrebt.

Der transpulmonale Druck ermöglicht es, die jeweiligen Anteile des Thoraxs und der Lunge an der Gesamtcompliance des Lungen-Thorax-Systems zu ermitteln. Er ist am Ende der Exspiration am kleinsten und am Ende der Inspiration am grössten. Da eine direkte Messung des Pleuradrucks nur über eine Pleurapunktion möglich wäre, wird in der Regel stellvertretend der ösophageale Druck mit Hilfe einer Ösophagussonde gemessen.

Doch auch diese Methode besitzt Nachteile, da die absoluten Werte des ösophagealen Drucks von zahlreichen Faktoren abhängig sind.

Dazu zählen unter anderem die jeweilige Atemmechanik, das Lungenvolumen, das Gewicht von Mediastinum und Abdomen, die Haltung des Patienten, die Reaktionsfähigkeit der glatten Muskulatur und die mechanischen Eigenschaften sowie der Füllungszustand des eingesetzten Ballons. Zudem erfordert Platzierung des Katheters und anschliessende Interpretation der gemessenen Werte bei starker Abhängigkeit von Lage und Messort sowie Beeinträchtigungen durch kardiale Artefakte ein hohes Mass an Kompetenz. Beispielsweise führt eine zu geringe Blockung des Ballons zu inkorrekter Druckübertragung, sodass die Drücke falsch als zu hoch gemessen werden.

Grundlage einer lungenprotektiven Beatmungsstrategie ist es, die Lunge möglichst wenig mechanischem Stress auszusetzen und dieser Protektion den Vorrang vor bester Oxygenierung und Ventilation zu lassen. Beatmung mit einem kleineren pro Atemzug applizierten Atemvolumen VT verursacht weniger beatmungsassoziierte Lungenschäden und verbessert das Überleben von Patienten, die an einem akuten Lungenversagen (ARDS «Acute Respiratory Distress Syndrome») leiden, signifikant.

Um das Gewebe zu schonen, wird zur Lungenprotektion die Höhe des positiven endexspiratorischen Drucks (PEEP «Positive End Expiratory Pressure») derart gewählt, dass er zu einer Reduktion der Druckamplitude während mechanischer Beatmung führt. Mit PEEP wird der Druck bezeichnet, der am Ende der Exspiration in der Lunge besteht. Im Atemzyklus markiert er den geringsten Druckwert.

Die Einstellung des individuell optimalen PEEP erfolgt nach Talmor im Rahmen des transpulmonalen Druckmonitorings. Hierbei wird ein ösophagealer Katheter gelegt, über den eine Messung des regionalen ösophagealen Drucks im Bereich um den Katheter ermöglicht wird. Durch Subtraktion dieses Druckes vom globalen Atemwegsdruck (P_{AW}) kann der transpulmonale Druck TPP berechnet werden.

Der PEEP wird zur Vermeidung von Atelektasen derart gewählt, dass ein resultierender endexspiratorischer TPP-Wert zwischen 0 und 10 mbar erreicht wird, der dabei möglichst knapp oberhalb von 0 mbar liegt, jedoch keine negativen Werte einnimmt. Andererseits wird zur Begrenzung von Spannungen in der einzelnen Alveole das pro Atemzug applizierte Atemvolumen VT so eingestellt, dass endinspiratorische TPP-Werte stets kleiner als 25 mbar sind.

Die elektrische Impedanztomographie (EIT) ist ein nichtinvasives bildgebendes Verfahren, das auf der Anwendung von Strom und der Messung der Spannung durch am Körper eines Patienten angebrachte Elektroden beruht. Die EIT liefert Bilder der Verteilung von elektrischer Leitfähigkeit, Admittivität, Impedanz oder Widerstand bzw. deren Veränderungen. Die gemessenen Werte werden in der Regel als EIT-Werte, Impedanzwerte oder Impedanzmesswerte bezeichnet. Diese Verteilung wird auch als "elektrische Eigenschaften" bezeichnet.

Das Bild wird als EIT-Bild bezeichnet. Das Bild oder die Bildsequenzen zeigen Unterschiede in den elektrischen Eigenschaften verschiedener Körpergewebe, Knochen, Haut, Körperflüssigkeiten und Organe, insbesondere der Lunge, die für die Überwachung des Zustands des Patienten nützlich sind.

Eine typische EIT-Anordnung ist in der WO 2015/048917 A1 dargestellt. Eine Reihe von Elektroden ist an einem Gürtel so angeordnet, dass sie in einem bestimmten Abstand voneinander um die Brust des Patienten herum angebracht sind und in elektrischem Kontakt mit der Haut stehen. Ein elektrisches Strom- oder Spannungseingangssignal wird abwechselnd zwischen verschiedenen oder allen möglichen Elektrodenpaaren angelegt. Während das Eingangssignal an eines der Elektrodenpaare angelegt wird, können die Ströme oder Spannungen zwischen den übrigen Elektroden gemessen werden. Die gemessenen elektrischen Spannungen des Körperabschnitts können durch einen Rekonstruktionsalgorithmus in elektrische Eigenschaften oder Änderungen der elektrischen Eigenschaften rekonstruiert werden. Diese können von einem Datenprozessor verwendet werden, um eine Darstellung der Verteilungsimpedanzwerte über einen Querschnitt des Patienten zu erhalten, um den der Elektrodengürtelring gelegt ist. Die elektrischen Eigenschaften werden auf einem Bildschirm angezeigt.

Beim EIT-Verfahren wird eine Reihe von Impedanzmesswerten an beispielswese 16, 32 oder mehr Elektroden aufgezeichnet. Aus diesen Impedanzmesswerten kann der EIT-Bildrekonstruktionsalgorithmus dann zweidimensionale Bilder mit Pixeln erzeugen, die Eigenschaften der Lunge darstellen. Das Bild kann aus 32 x 32 Pixeln bestehen, die Stellen innerhalb und ausserhalb der Lunge darstellen.

Die durch EIT gewonnenen Informationen über elektrische Eigenschaften können in ein Querschnittsbild projiziert werden, das von einem anatomischen Modell in einem anatomischen Kontext abgeleitet ist und beispielsweise Konturen zeigt, die die äusseren Grenzen der modellierten Organe innerhalb der Elektrodenebene darstellen.

Konturen funktioneller Strukturen können dazu verwendet werden, die Pixel innerhalb solcher Strukturen (automatisch) zu clustern, um sogenannte «Regions of Interest» (ROI) zu bilden, die mit funktionell bedeutsamen anatomischen Strukturen wie der Lunge übereinstimmen. Signale von Pixeln, die in solche ROIs fallen, können durch automatische Signalverarbeitungsmittel und -algorithmen identifiziert und für jedes der ROIs separat analysiert werden.

Es können bis zu 100 oder sogar mehr tomographische EIT-Bilder pro Sekunde rekonstruiert werden. Diese zeitlich hochauflösenden Bilder spiegeln die regionalen elektrischen Eigenschaften im Lungengewebe wider, die durch die Atem- und Herzzyklen beeinflusst werden. Im Gegensatz zu CT-Scans zeigen EIT-Bilder keine morphologischen, sondern funktionelle Informationen, wie regionale Tidalventilation, lokale Lungenrekrutierung, exspiratorische Zeitkonstanten oder die Verteilung der Lungenperfusion. Die von der EIT gemessenen Daten korrelieren mit den entsprechenden Lungenvolumina auf regionaler oder Pixelebene.

Scaramuzzo et al. (Scaramuzzo G, Spandaro S, Spinelli E, Saldmann AD, Bohm SH, Ottaviani I, Montanaro F, Gamberini L, Marangoni E, Amuri T and Volta CA (2021) Calculation of Transpulmonary Pressure From Regional Ventilation Displayed by Electrical Impedance Tomography in Acute respiratory Distress Syndrome. Front Physiol. 12:693736. doi: 10.3389/fphys.2021.693736) schlagen vor, die Beziehung zwischen regionalen mit der EIT gemessenen Parametern heranzuziehen, um die Differenz zwischen dem endinspiratorischen und dem endexspiratorischen transpulmonalen Druck (DP_{L}) nicht invasiv zu bestimmen.

Strodthoff et al. (Strodthoff N, Strodthoff C, Becher T, Weiler N, Frerichs I. arXiv:2010.09622v1 [eess.IV] 19. Oct 2020) leiten mittels AI aus gemessenen EIT Daten gewisse physiologische Parameter ab. Unter anderem soll aus einer Kombination von EIT-Daten und absolutem Atemwegsdruck, also nicht invasiv, auf den ösophagealen Druck geschlossen werden.

Die mit den Verfahren von Scaramuzzo et al. und Strodthoff et al. ermittelten Werte sind in der Regel für eine differenzierte Beatmungseinstellung eines individuellen Patienten zu ungenau.

Becher et al. (Ann. Intensive Care (2021) 11:89) haben ein Protokoll entwickelt, um den PEEP Wert und das Tidalvolumen auf Grundlage von EIT-basierten Daten zu individualisieren. Das Protokoll ersetzt allerdings nicht das transpulmonale Druckmonitoring mittels Ösophagussonde.

Es besteht die Aufgabe, eine Vorrichtung, ein System, ein Verfahren sowie ein Computerprogramm zur Verfügung zu stellen, welche die Nachteile des Bekannten überwinden, mittels welchen insbesondere möglichst genau eine Grösse, die repräsentativ für den transpulmonalen Druck ist, bestimmt werden kann, ohne einen invasiven Eingriff am Patienten vorzunehmen, insbesondere ohne eine Ösophagussonde setzen zu müssen.

Die vorstehende Aufgabe wird mit einer Vorrichtung, einem System, einem Verfahren sowie einem Computerprogramm und einem Computerprogrammprodukt gemäss den unabhängigen Ansprüchen gelöst.

Bei der Vorrichtung handelt es sich um eine Vorrichtung zur nicht invasiven Bestimmung einer Grösse, die repräsentativ für den Verschlussdruck ist. Der Verschlussdruck ist der Atemwegsdruck, bei welchem der exspiratorische transpulmonale Druck kleiner gleich null wird.

Die Vorrichtung umfasst einen Eingang für Daten eines Beatmungsdrucks von einem Beatmungsgerät über mindestens einen Beatmungszyklus. Das Beatmungsgerät erzeugt über den Beatmungszyklus ein kontrolliertes Profil mit einer ansteigenden und einer nachfolgenden absteigende Druckrampe. Der Eingang dient zum Empfangen der Daten des Druckprofils.

Das Druckprofil kann auch von einem anderen geeigneten Apparat erzeugt werden und die Daten von diesem Apparat am Eingang empfangen werden.

Dabei könnte es sich um ein Gerät handeln, welches einen Atemgasfluss generiert, zum Beispiel ein Flowmeter, welches dabei den jeweils herrschenden Druck misst und welches dann entsprechend schrittweise den Fluss steigert, um den gewünschten Druck zu erzielen.

Es könnte aber auch eine eigens dafür entwickelte Vorrichtung sein, die eine Gasquelle mit einem Drucksensor sowie einem Controller verbindet, der diese Rampe dann steuert oder regelt.

Das Druckprofil kann bei einem zuvor festgelegten PEEP oder bei einem ZEEP ("zero endexpiratory pressure") beginnen, und, insbesondere linear über die Zeit, über die Eröffnungs- oder Rekrutierungsdrücke hinaus bis zu einem Maximum gefahren wird. Nach Erreichen des Maximums kann der Druck rampenartig, insbesondere linear über die Zeit, gesenkt werden, bis er das bisherige PEEP-Niveau oder aber ZEEP erreicht hat.

Die Vorrichtung umfasst ausserdem einen Eingang für EIT-Daten von einer EIT-Vorrichtung, die während des mindestens einen Beatmungszyklus erhoben wurden. Diese Daten können am Eingang für EIT-Daten von der Vorrichtung empfangen werden.

Die EIT-Vorrichtung kann Daten liefern, die zum Beispiel Information über lokale Impedanzen oder Leitfähigkeiten enthalten.

Als EIT-Werte werden die Änderungen der EIT-Daten, insbesondere der festgestellten Impedanzen oder Leitfähigkeiten, gegenüber einer Baseline, bzw. einem Referenzwert, bezeichnet.

EIT-Bilder können als Impedanz oder Leitfähigkeitsbilder dargestellt werden. Bei den EIT-Werten kann es sich somit grundsätzlich um Impedanzänderungen oder um Leitfähigkeitsänderungen handeln.

Die EIT-Werte, die zum Beispiel als regionale EIT-Werte pixelweise erhoben werden können, korrelieren mit den entsprechenden Lungenvolumina.

Die Vorrichtung umfasst zudem eine Recheneinheit, die dazu ausgelegt ist, auf Grundlage der Daten des Beatmungsgeräts und der Daten des EIT-Geräts eine erste zeitliche Ableitung der EIT-Werte über mindestens einen Beatmungszyklus zu bestimmen und in Relation zum Beatmungsdruck zu setzen.

Die Bestimmung kann pixelweise oder über mehrere Pixel, insbesondere über eine Zeile oder Spalte oder über alle relevanten Pixel, erfolgen.

Als relevante Pixel können Pixel betrachtet werden, welche Volumina darstellen, die zur Belüftung der Lunge beitragen.

Werden die EIT-Werte nach der Zeit abgeleitet, so resultieren daraus Werte, die die Änderung der genannten EIT-Werte im Verlauf der Zeit anzeigen. Letztere korrelieren mit dem Volumenstrom oder dem Fluss der Atemgase.

Insbesondere auf regionaler Ebene kann mittels EIT die erste zeitliche Ableitung der EIT-Werte, insbesondere die zeitliche Ableitung der Impedanz jedes Pixels, als Surrogatparameter für den regionalen Fluss dV/dt in dieser Lungenregion herangezogen werden.

Die Recheneinheit ist dazu ausgelegt, zumindest eine charakteristische Eigenschaft der Kurve der ersten zeitlichen Ableitung der EIT-Werte gegenüber dem Beatmungsdruck zu bestimmen. Diese charakteristische Eigenschaft ist repräsentativ für den Verschlussdruck, also den Atemwegsdruck, bei dem der transpulmonale Druck kleiner gleich null wird.

Mit Erhöhung des Atemwegsdruckes fliesst Luft in die Lungen hinein. Das Volumen kann zunächst ungefähr proportional zum Druckanstieg ansteigen, so dass die Flusskurve in etwa parallel zur Druckachse verlaufen kann. Sobald zusätzliche Lungenareale, die zuvor nicht zugänglich waren, eröffnet werden (Recruitment), nimmt der Fluss überproportional zu. Dieses Eröffnen kann schlagartig geschehen. Solange weitere Lungenareale rekrutiert werden, bleibt der Fluss hoch. Werden keine weiteren Lungenareale durch den ansteigenden Druck erschlossen, kann der Fluss wieder parallel zur Druckachse verlaufen. Eine weitere Lungeneröffnung findet nicht statt. Dieser Fluss kann aufgrund des nun durch die vorherige Rekrutierung erhöhten Lungenvolumens gegenüber dem anfänglichen Fluss erhöht, also parallel verschoben, sein.

Die erschlossenen Lungenareale werden mit weiterem Druck nur weiter befüllt. Daher ist eine weitere Erhöhung des Druckes nicht mehr zielführend.

Der Druck kann daher wieder gesenkt werden. Dabei strömt Gas aus den Lungen. Dies generiert einen negativen Fluss, der zunächst konstant ist. Die Flusskurve verläuft demnach wieder parallel zur Druckachse.

Erreicht der Druck Werte, die zur Stabilisierung der Alveolen nicht mehr ausreichen, beginnen die Alveolen zu kollabieren, was plötzlich geschehen kann. Bei dem zumeist plötzlichen Lungenkollaps strömt überproportional viel Gas aus den Lungen.

Das macht sich in einer Zunahme des negativen Flusses bemerkbar.

Kollabiert mit weiterer Drucksenkung weiteres Lungengewebe, bleibt der Fluss hoch und ebbt erst wieder ab, wenn der Kollaps-Prozess abgeschlossen ist.

Der Fluss verläuft dann wieder parallel zur Druckachse.

Da die erste zeitliche Ableitung der EIT-Werte der zeitlichen Änderung des Lungenvolumens entspricht, verläuft die Kurve der ersten zeitliche Ableitung der EIT-Werte über den Beatmungsdruck analog zum Fluss.

Gemäss einer bevorzugten Ausführungsform der Erfindung kann als charakteristische Eigenschaft der Kurve der ersten zeitlichen Ableitung der EIT-Werte insbesondere mindestens ein charakteristischer er Kurvenpunkt bestimmt werden.

Exspiratorische Kurvenpunkte liegen in einem Druckbereich, der beginnt, wenn der Druck abgesenkt wird, und der typischerweise endet, wenn der PEEP-Wert erreicht ist. Die Alveolen können im Verlauf der Exspiration kollabieren.

Die zu bestimmenden charakteristischen exspiratorischen Kurvenpunkte sind charakteristisch für das, insbesondere beginnende, Kollabieren der Alveolen, das typischerweise auftritt, wenn der exspiratorische transpulmonale Druck negativ wird.

Da der Atemwegsdruck aus der Beatmungsrampe bekannt ist, kann somit auf den ösophagealen Druck zum Zeitpunkt des Alveolenkollapses geschlossen werden.

Insbesondere kann der Deflektionspunkt, der Wendepunkt und/oder das Minimum, der ersten zeitliche Ableitung der EIT-Werte in Abhängigkeit des Beatmungsdrucks zu bestimmt werden.

Bezüglich der gesamten Lunge liegt der charakteristische exspiratorische Kurvenpunkt zwischen dem Beginn des Kollapses, also dem Punkt, an dem der Kurve von der parallelen Geraden abweicht (Deflektionspunkt), und dem negativen Flussmaximum, bei dem der Fluss und damit der Kollaps in vollem Gange sind. Letzterer ist eher zu spät, während ersterer eher früh ist.

Man kann diese charakteristischen exspiratorischen Kurvenpunkte durch eine weitere Ableitung der Flusskurve nach der Zeit, also mittels Tangentenbildung, bestimmen.

Der Wendepunkt der exspiratorischen Kurve, wo die Steigung der Ableitung am größten ist, liegt zwischen dem Deflektionspunkt und dem Maximum. Der Druck an diesem Punkt kann dem gesuchten Druckwert am nächsten kommen.

Die EIT-Werte können zeitlich abgleitet und über dem Beatmungsdruck aufgetragen werden. An dieser Kurve können mittels üblicher rechnerunterstützter Methoden Extrema, Wendepunkte und Grenzwertüberschreitungen festgestellt werden.

Die EIT-Signale sind im Allgemeinen überlagert von kardiogenen (herzschlagbedingten) oszillierenden Signalen.

Die Recheneinheit kann dazu ausgelegt sein, die kardiogenen Signale aus den EIT-Daten der EIT-Vorrichtung herauszufiltern, um die ventilationsbedingten Signale zu extrahieren.

Die Recheneinheit kann dazu dafür ausgelegt sein, die Daten über einen an die Herzfrequenz angepassten Filter, zum Beispiel einen Tiefpassfilter, einen Bandpass oder einen Bandstop, zu leiten, in welchem bevorzugt Frequenzen der ein- bis vierfachen Herzfrequenz aus dem EIT-Signal gefiltert werden, also Frequenzen im Bereich von 0-0.2Hz.

Die Recheneinheit kann dazu ausgelegt sein, einen PEEP-Wert auf Grundlage der charakteristischen Eigenschaft der Kurve festzulegen, insbesondere auf Grundlage des charakteristischen exspiratorischen Kurvenpunkts.

Diejenigen Pixel, die im Bereich des Ösophagus liegen, sollten kollabieren, wenn der transpulmonale Druck unter Null fällt. Der zu diesem Zeitpunkt im Ösophagus herrschende Atemwegsdruck entspricht dann genau dem PEEP, der nötig wäre, um diesen Kollaps zu kompensieren.

Der Druck bei den charakteristischen Kurvenpunkten, insbesondere der Druck, bei dem die ersten Anzeichen von Kollaps festgestellt werden, ist derjenige, bei dem der exspiratorische transpulmonale Druck kleiner gleich Null wird. Dieser Druck entspricht dann dem PEEP, der nötig wäre, um den Kollaps zu kompensieren, und kann demnach als geeigneter einzustellender PEEP-Wert betrachtet werden.

Die Recheneinheit kann dazu ausgelegt sein, die zeitliche Ableitung der EIT-Werte regional zu bestimmen, insbesondere über einen oder mehrere ausgewählte Einzelpixel, weiter insbesondere über eine Pixelzeile.

So können die zeitlichen Ableitungen der EIT-Werte, insbesondere der Impedanzänderungen, aller Pixel eines EIT-Bildes berechnet und deren Verlauf quer zum Gravitationsvektor entweder in Einzelpixeln oder aber für ganze Pixelzeilen zusammen interpretiert werden.

Der Schwerkraftvektor macht sich bei einem auf dem Rück liegenden Patienten als zunehmender Druckgradient von vorne nach hinten in der liegenden Lunge bemerkbar.

Es können alle Pixel in den jeweiligen Zeilen, die senkrecht zum Schwerkraftvektor liegen, aufsummiert und analysiert werden.

Von besonderem Interesse ist dabei der Flussverlauf in der Pixelzeile des EIT-Bildes, die horizontal durch den Ösophagus läuft, denn deren Verhalten sollte die Situation im Ösophagus widerspiegeln.
Nimmt in diesen Pixeln der EIT-Wert bei Druckreduktion schlagartig ab, so kann daraus geschlossen werden, dass der in den entsprechenden Alveolen herrschende Druck nicht mehr ausreicht, einen am Kollaps zu verhindern. Dieser Punkt kann somit als Zeichen für den Übergang von offenen zu kollabierten Alveolen gewertet werten, was einer Situation entspricht, bei welcher der mit einer Ösophagussonde ermittelte transpulmonalen Druck einen Wert von um die Null erreicht.

Die Recheneinheit kann dazu ausgelegt sein, eine Beatmungsrampe festzulegen. Einer lineare Beatmungsrampe ist charakterisiert durch den Startdruck, die positive Steigung, den Maximaldruck und den Enddruck.

Die Druckrampe kann in der Regel bei 0 mbar starten und einen linearen Anstieg um 1 mbar/s zeigen. Nach Erreichen des Maximums kann die Entlastung der Druckrampe um wiederum 1 mbar/s erfolgen.

Das Maximum sowie der Enddruck können zuvor absolut festgelegt, oder aber auch aus den gemessenen Daten im Verlaufe des Verfahrens abgeleitet werden.

Die Vorrichtung kann einen Ausgang zum Abgeben von Daten an das Beatmungsgerät umfassen, insbesondere Daten für eine Beatmungsrampe und/oder Daten für einen bestimmten PEEP-Wert. Die Vorrichtung ist dann als Vorrichtung zur Steuerung eines Beatmungsgeräts ausgebildet.

Die Recheneinheit kann dazu ausgelegt sein, die charakteristische Eigenschaft der Kurve und insbesondere den PEEP-Wert vor einer Beatmung und/oder während der Beatmung, insbesondere regelmässig, zu bestimmen.

So kann auch während einer längeren Beatmung immer ein aktuell angepasster PEEP-Wert festgelegt und eingestellt werden.

Die Vorrichtung kann über eine Anzeigeeinheit, zum Beispiel ein Display, verfügen, mit welcher die Kurven und/oder die festgelegten Werte angezeigt werden. Die Vorrichtung kann auch eine Ausgabeeinheit umfassen, über welche Kurvendaten und/oder die festgelegten Werte an eine externe Anzeigeeinheit abgegeben werden.

Diesel Wert kann auch direkt an ein Beatmungsgerät ausgegeben werden, damit dann damit der PEEP nachgeregelt wird.

Die Aufgabe wird ausserdem gelöst durch ein System umfassend ein EIT-Gerät, ein Beatmungsgerät und eine Vorrichtung wie oben beschrieben.

Die Vorrichtung kann integraler Bestandteil des EIT-Geräts und/oder des Beatmungsgeräts sein.

Die Aufgabe wird ausserdem gelöst durch ein Verfahren zum Bestimmen von Grössen, die repräsentativ für den Verschlussdruck sind, also des Atemwegsdrucks, bei welchem der exspiratorische transpulmonale Druck kleiner gleich null wird.

Dabei werden Werte eines Beatmungsdrucks von einem Beatmungsgerät über mindestens einen Beatmungszyklus empfangen. Ausserdem werden EIT-Daten von einer EIT-Vorrichtung empfangen, die während des mindestens einen Beatmungszyklus erhoben werden.

Eine erste zeitliche Ableitung von EIT-Werten wird in Abhängigkeit des Beatmungsdrucks über mindestens einen Beatmungszyklus auf Grundlage der Daten des Beatmungsgeräts und des EIT-Geräts bestimmt.

Zumindest eine charakteristische Eigenschaft der Kurve der ersten zeitlichen Ableitung der EIT-Werte in Abhängigkeit des Beatmungsdrucks wird bestimmt.

Die charakteristische Eigenschaft ist repräsentativ für den Verschlussdruck.

Insbesondere wird als charakteristische Eigenschaft mindestens ein charakteristischer exspiratorischer Kurvenpunkt bestimmt.

Insbesondere wird der Deflektionspunkt, der Wendepunkt und/oder das Minimum der ersten zeitlichen Ableitung der EIT-Werte in Abhängigkeit des Beatmungsdrucks bestimmt.

Das Verfahren wird insbesondere mit einer Vorrichtung, wie sie oben beschrieben ist, durchgeführt.

Während der langsamen Druckveränderungen kann gleichzeitig und synchron das Atemwegsvolumen und/oder der Volumenstrom oder Fluss der Gase in den Atemwegen (dV/dt) über die Zeit mittels Gasflusssensor gemessen werden.

Damit kann sichergestellt werden, dass sich der Verlauf der globalen Atemgas-Flusskurve und des globalen EIT-Werts (der aus allen Pixeln zusammengesetzt ist) sehr ähnlich sind.

Bei einer linearen Beatmungsrampe zeigt sowohl der Verlauf des Atemwegvolumens als auch der Verlauf der EIT-Werte ein glockenförmiges Muster. Dabei kommt es zunächst zu einem steilen Anstieg des Signals bei Beginn der Rampe, um im Verlauf in Richtung des Maximums etwas abzuflachen. Nachdem das Maximum durchlaufen wurde, nimmt das Signal zunächst geringfügig und schliesslich immer stärker ab, um auf einem höheren Niveau als zu Beginn der Rampe zu enden.

Der PEEP-Wert kann auf Grundlage des charakteristischen exspiratorischen Kurvenpunkts festgelegt werden.

Der PEEP-Wert kann als Steuergrösse an das Beatmungsgerät ausgegeben werden. Die Beatmung kann auf Grundlage dieses PEEP-Werts durchgeführt werden.

Das Verfahren kann vor einer Beatmung und/der während der Beatmung, insbesondere regelmässig, durchgeführt werden.

Das Verfahren kann beispielsweise in vorbestimmten Zeitabständen oder nach einer vorbestimmten Anzahl von Atemzügen durchgeführt werden.

Die Aufgabe wird ausserdem gelöst durch ein Computerprogramm mit Programmcode zur Durchführung der Schritte des Verfahrens wie oben beschrieben, wenn das Programm auf einem Computer ausgeführt wird.

Die Aufgabe wird ausserdem gelöst durch ein Computerprogrammprodukt, das direkt in einen internen Speicher eines Digitalcomputers geladen werden kann und das Softwarecodeabschnitte umfasst, die die Verfahrensschritte, wie sie oben beschrieben sind, ausführen, wenn das Programm auf dem Digitalcomputer läuft.

Die Erfindung wird nachfolgend anhand von Zeichnungen erläutert. Dabei zeigen
- Fig. 1: den schematischen Aufbau eines Systems, umfassend eine erfindungsgemässe Vorrichtung, ein EIT-Gerät und ein Beatmungsgerät;
- Fig. 2: den zeitlichen Verlauf von Beatmungsdruck und Fluss;
- Fig. 3: den Fluss aufgetragen gegenüber dem Beatmungsdruck;
- Fig. 4: die erste zeitliche Ableitung eines EIT-Werts in Abhängigkeit des Beatmungsdrucks für eine gesunde Lunge;
- Fig. 5: die erste zeitliche Ableitung eines EIT-Werts in Abhängigkeit des Beatmungsdrucks für eine beeinträchtigte Lunge;
- Fig. 6: eine Gegenüberstellung eines CT-Bildes und eines EITBildes;
- Fig. 7: die ersten zeitlichen Ableitungen der regionalen EITWerte in Abhängigkeit des Beatmungsdrucks für eine rechte und eine linke Lunge.

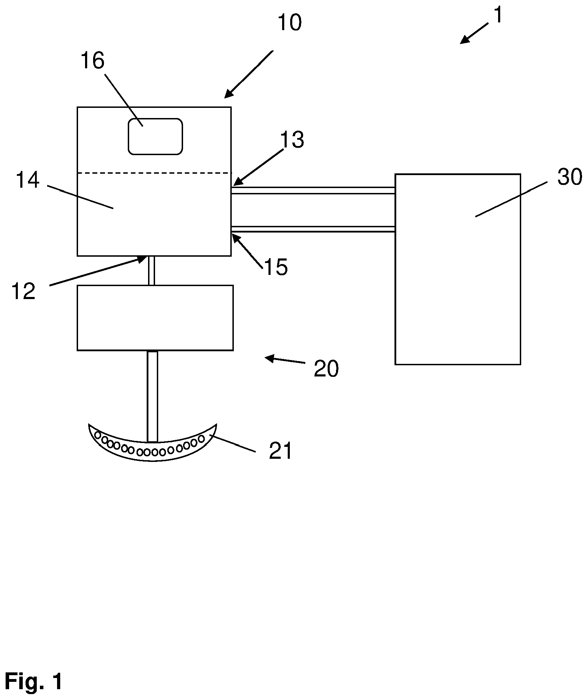

Figur 1 zeigt ein System 1 mit einem EIT-Gerät 20 und einem Beatmungsgerät 30.

Das EIT-Gerät 20 umfasst einen Elektrodengürtel 21, der an einen Patienten angebracht werden kann und mit dem Daten für ein EIT-Bild erfasst werden können.

Das System 1 umfasst ausserdem eine Vorrichtung 10 zur nicht invasiven Bestimmung einer Grösse, die repräsentativ für den Verschlussdruck ist, also für den Atemwegsdruck, bei welchem der exspiratorische transpulmonale Druck kleiner gleich Null wird.

Die Vorrichtung 10 weist einen Eingang 13 zum Empfangen von Daten eines Beatmungsdrucks von dem Beatmungsgerät 30 über mindestens einen Beatmungszyklus auf.

Die Vorrichtung 10 weist ausserdem einen Eingang 12 zum Empfangen von EIT-Daten von dem EIT-Gerät 20 auf, die während des mindestens eines Beatmungszyklus erhoben wurden.

Die Vorrichtung 10 weist zudem eine Recheneinheit 14 auf, die dazu ausgelegt ist, auf Grundlage der Daten des Beatmungsgeräts 30 und des EIT-Geräts 20 die erste zeitliche Ableitung von EIT-Werten in Abhängigkeit des Beatmungsdrucks über mindestens einen Beatmungszyklus zu bestimmen.

Die Recheneinheit ist ausserdem dazu ausgelegt, zumindest eine charakteristische Eigenschaft der Kurve der ersten zeitliche Ableitung der EIT-Werte in Abhängigkeit des Beatmungsdrucks zu bestimmen. Insbesondere wird ein charakteristischer exspiratorischer Kurvenpunkt bestimmt.

Die Vorrichtung 10 umfasst ein Display 16 zum Anzeigen der Kurve und/oder der bestimmten Werte.

Die Vorrichtung umfasst einen Ausgang 15 zum Abgeben von Daten an das Beatmungsgerät 30.

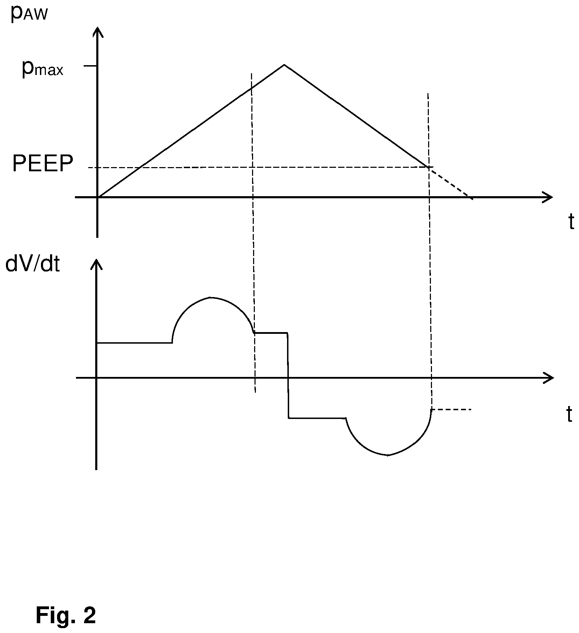

Der Atemwegsdruck p_{AW} ist in Figur 2 synchron mit dem Fluss dV/dt über die Zeit t abgetragen. Die Flusskurve dV/dt zeigt während der Druckerhöhung eine charakteristische Konvexität, die das Eröffnen von Lungenarealen anzeigt. Bei Druckerniedrigung ist eine Konkavität zu erkennen, welche das Kollabieren von Lungeneinheiten anzeigt.

Der Atemwegsdruck p_{AW} wird nur bis zu einem vorherbestimmten PEEP-Wert reduziert.

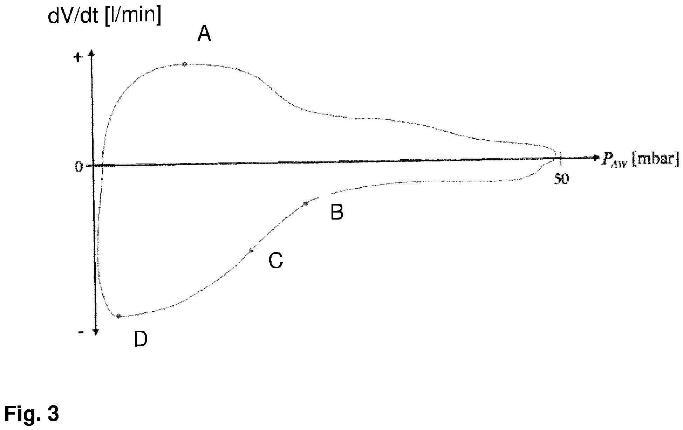

Figur 3 zeigt den Fluss dV/dt aufgetragen gegenüber dem Beatmungsdruck p_{Aw}. Die Kurve weist einige charakteristische Punkte auf.

Der Fluss kann mit einem Gasflusssensor gemessen werden. Diese Messung ist nicht Teil des erfindungsgemässen Verfahrens.

Auf dem Weg der Druckerhöhung (oberer Teil der Kurve) wird ein positives Flussmaximum A erreicht. Bei der Reduzierung des Drucks wird zunächst ein Deflektionspunkt B, dann ein Wendepunkt C und anschliessend ein negatives Flussmaximum D erreicht.

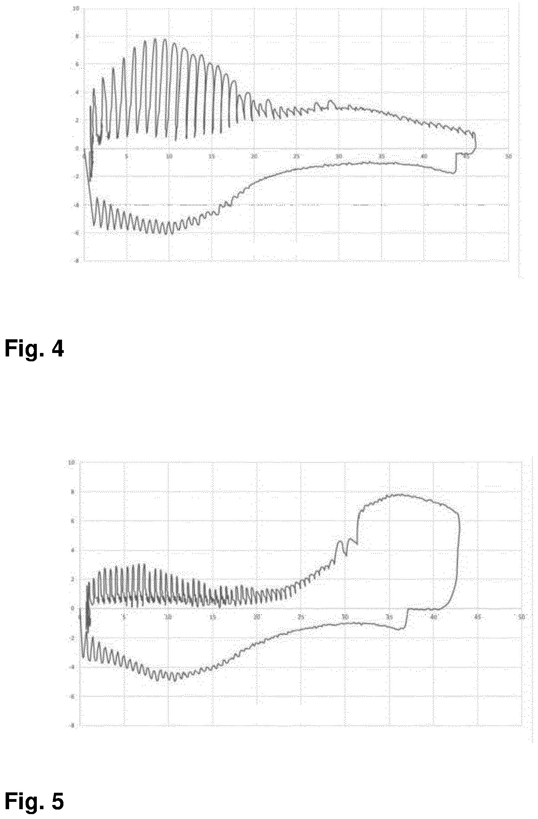

Figur 4 zeigt eine ungefilterte Originalkurve während der Durchführung des erfindungsgemässen Verfahrens. Dargestellt ist die erste zeitliche Ableitung von EIT-Werten, hier einer relativen Impedanzänderung, in Abhängigkeit des Beatmungsdrucks, die an einer Lunge eines gesunden Schweines gemessen wurde, in diesem Fall summiert über alle relevanten Pixel.

Es ist zu erkennen, dass der Kurvenverlauf prinzipiell den in Figuren 2 und 3 gezeigten Flusskurven entspricht. Es kann von einer Korrelation zwischen der zeitlichen Ableitung der EIT-Werte über alle relevanten Pixel und dem globalen Atemwegsfluss dV/dt ausgegangen werden.

Die überlagerten herzschlagsynchronen Oszillationen sind zu erkennen.

Eine Eröffnung der Lungen findet in diesem Fall nicht statt, da diese Atemwegsdrücke von über 25 mbar voraussetzt.

Die in Figur 2 genannte Konvexität ist jedoch bei absteigendem Druck zu erkennen. Diese deutet einen gewissen Lungenkollaps an, der sein Maximum bei ca. 10 mbar erreicht.

Figur 5 zeigt die entsprechende Kurve, die bei einem Schwein gemessen wurde, bei welchem ein zweistündiges "ventilator induced lung injury" (VILI) induziert wurde, sodass die Lunge einer Lunge in einem ARDS-Zustand entspricht.

Um den ARDS-artigen Zustand des Lungenschadens zu untersuchen, wird ein sogenanntes zweistufiges Surfactant-Lavage-Modell angewandt. Im Anschluss an die erste Phase der Messungen im lungengesunden Zustand werden die Lungen der Tiere wiederholt über einen endotrachealen Tubus mit 35 ml/kg KG körperwarmer, isotonischer Natriumchlorid (NaCl)-Lösung 0.9 % lavagiert. Das Surfactant wird dadurch aus den Lungen herausgewaschen, bis eine optisch klare und nicht mehr schäumende Lavagelösung entsteht.

Nach Bestätigung dieser notwendigen Bedingung erfolgt eine 120 min andauernde, lungenschädigende Beatmung mit einer inspiratorischen Sauerstofffraktion von 1.0, einem PEEP von 0 mbar und Tidalvolumen von 15 ml/kg bei einer Atemfrequenz von 12 Atemzügen pro Minute und Inspiration zu Exspiration-Verhältnis von 1:2.

Während der Druckerhöhung bleibt die erste zeitliche Ableitung der EIT-Werte, die dem Fluss entspricht, geringer als bei dem gesunden Schwein. Der Fluss steigt erst an, wenn sich die Lunge zu eröffnen beginnt. Das Maximum der Konvexität liegt bei ca. 37 mbar. Die Eröffnung von Lungenarealen ist deutlich zu sehen, bleibt aber unvollständig, da das Manöver bei ca. 42 mbar in dieser konkreten Messung versehentlich abgebrochen wurde. Das Maximum des Alveolarkollapses liegt bei ca. 10 mbar.

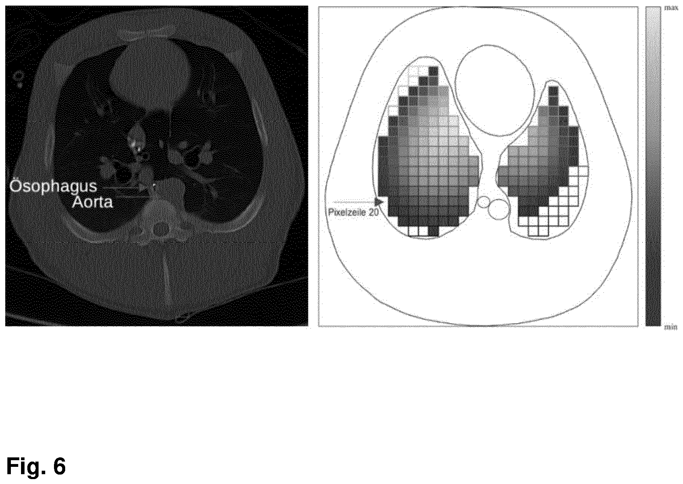

Figur 6 zeigt eine Gegenüberstellung eines CT-Bildes und eines EIT-Bildes. Das EIT-Bild ist ein Tidalbild und zeigt die EIT-Werte, in diesem Fall Impedanzänderungen, für jedes Pixel in der Messebene, die im CT-Bild dargestellt ist.

Es wird angenommen, dass das regionale Pixel-Impedanzsignal mit dem regionalen Volumen innerhalb dieses Pixels korreliert.

Das EIT-Bild kann pixelweise oder zeilenweise ausgewertet werden. Im vorliegenden Fall entspricht die Pixelzeile 20 der Höhe des Ösophagus.

Pixel des EIT-Bildes, die weniger als 10% der maximalen Impedanzamplitude dieser Lungenregion ausmachen, werden aus der Auswertung der regionalen Kurven ausgeschlossen. Sie stellen die sogenannten «silent spaces» dar.

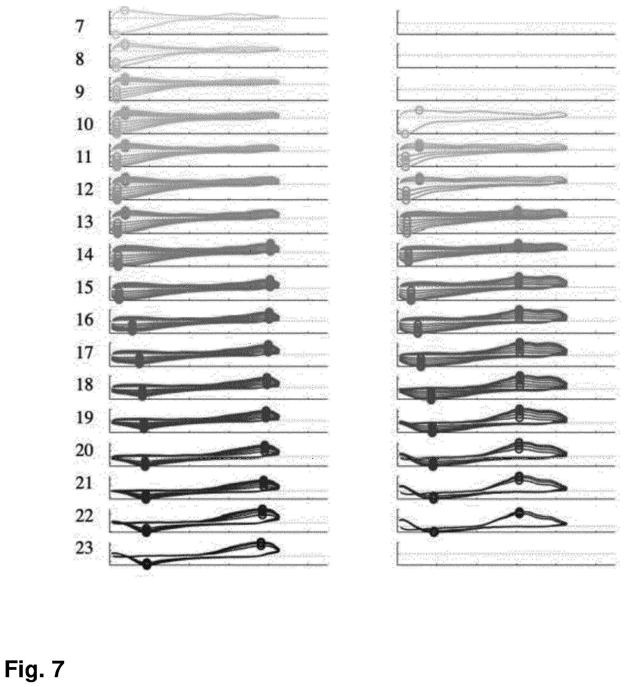

Figur 7 zeigt die ersten zeitlichen Ableitungen der regionalen EIT-Werte in Abhängigkeit des Beatmungsdrucks für die Zeilen der rechten und der linken Lunge.

Für jede Pixelzeile sind die Ergebnisse der jeweiligen Pixel aufgezeigt. Zudem sind die Maxima der Kurven gekennzeichnet.

Es hat sich gezeigt, dass die negativen Maxima der Pixelzeile 20, die als Verschlussdruck bezeichnet werden können, mit dem parallel über eine Ösophagus-Sonde gemessenen PEEP nach Talmor korrelieren.

Auf Grundlage der EIT-Messung kann somit ein Wert, der charakteristisch für den Verschlussdruck ist, bestimmt werden.

Die Einstellung des individuell optimalen PEEP erfolgt nach Talmor im Rahmen des transpulmonalen Druckmonitorings. Hierbei wird ein ösophagealer Katheter gelegt, über den eine Messung des regionalen ösophagealen Drucks im Bereich um den Katheter ermöglicht wird. Durch Subtraktion dieses Druckes vom globalen Atemwegsdruck gelingt schliesslich eine Berechnung des transpulmonalen Drucks.

Der PEEP wird nun zur Vermeidung von Atelektasen derart gewählt, dass der resultierende endexspiratorische transpulmonale Druck zwischen 0 und 10 mbar erreicht und dabei möglichst knapp oberhalb von 0 mbar liegt, jedoch keine negativen Werte erzielt.

Andererseits wird zur Begrenzung von Spannungen in der einzelnen Alveole das Tidalvolumen so eingestellt, dass endinspiratorische TPP-Werte stets kleiner als 25 mbar liegen.

Die regionalen Verschlussdrücke können als nichtinvasiver Surrogatparameter für den transpulmonalen Druck nach Talmor für die PEEP-Einstellung genutzt werden.

## Patentansprüche

1. Vorrichtung (10) zur nicht invasiven Bestimmung einer Grösse, die repräsentativ für den Verschlussdruck ist, umfassend
- einen Eingang (13) für Daten eines Beatmungsdrucks von einem Beatmungsgerät (30) über mindestens einen Beatmungszyklus;
- einen Eingang (12) für EIT-Daten von einem EIT-Gerät (20), die während des mindestens eines Beatmungszyklus erhoben wurden;
- eine Recheneinheit (14), die dazu ausgelegt ist, auf Grundlage der Daten des Beatmungsgeräts und des EIT-Geräts die erste zeitliche Ableitung von EIT-Werten in Abhängigkeit des Beatmungsdrucks über mindestens einen Beatmungszyklus zu bestimmen und
die dazu ausgelegt ist, zumindest eine charakteristische Eigenschaft der Kurve der ersten zeitlichen Ableitung der EIT-Werte in Abhängigkeit des Beatmungsdrucks zu bestimmen, die repräsentativ für den Verschlussdruck ist, insbesondere einen charakteristischen exspiratorischen Kurvenpunkt, weiter insbesondere einen Deflektionspunkt, einen Wendepunkt und/oder ein Minimum.

2. Vorrichtung gemäss Anspruch 1, wobei die Recheneinheit dazu ausgelegt ist, einen PEEP-Wert auf Grundlage der charakteristischen Eigenschaft der Kurve, insbesondere des charakteristischen exspiratorischen Kurvenpunkts, festzulegen.

3. Vorrichtung gemäss Anspruch 1 oder 2, wobei die Recheneinheit (14) dazu ausgelegt ist, die erste zeitliche Ableitung der EIT-Werte regional zu bestimmen, insbesondere über einen oder mehrere ausgewählte Einzelpixel, weiter insbesondere über eine Pixelzeile.

4. Vorrichtung gemäss einem der vorhergehenden Ansprüche, wobei die Recheneinheit (14) dazu ausgelegt ist, eine Beatmungsrampe festzulegen.

5. Vorrichtung gemäss einem der vorhergehenden Ansprüche zur Steuerung eines Beatmungsgeräts, wobei die Vorrichtung einen Ausgang (15) zum Abgeben von Daten, insbesondere Daten für eine Beatmungsrampe und/oder Daten für einen bestimmten PEEP-Wert, an das Beatmungsgerät (30) umfasst.

6. Vorrichtung gemäss einem der vorhergehenden Ansprüche, wobei die Recheneinheit (14) dazu ausgelegt ist, die charakteristische Eigenschaft der Kurve und insbesondere den PEEP-Wert vor einer Beatmung und/oder während der Beatmung, insbesondere regelmässig, zu bestimmen.

7. System (1) umfassend ein EIT-Gerät (10), ein Beatmungsgerät (30) und eine Vorrichtung (1) gemäss einem der vorangehenden Ansprüche.

8. System gemäss Anspruch 7, wobei die Vorrichtung integraler Bestandteil des EIT-Geräts (20) und/oder des Beatmungsgeräts (30) ist.

9. Verfahren zum Bestimmen von Grössen, die repräsentativ für den Verschlussdruck sind, umfassend die Schritte
- Empfangen von Daten eines Beatmungsdrucks von einem Beatmungsgerät über mindestens einen Beatmungszyklus,
- Empfangen von EIT Daten von einem EIT-Gerät, die während des mindestens eines Beatmungszyklus erhoben wurden;
- Bestimmung einer ersten zeitliche Ableitung der EIT-Werte in Abhängigkeit des Beatmungsdrucks über mindestens einen Beatmungszyklus auf Grundlage der Daten des Beatmungsgeräts und des EIT-Geräts,
- Bestimmung zumindest einer charakteristisches Eigenschaft der Kurve der ersten zeitlichen Ableitung der EIT-Werte in Abhängigkeit des Beatmungsdrucks, die repräsentativ für den Verschlussdruck ist, insbesondere eines charakteristischen exspiratorischen Kurvenpunkts, weiter insbesondere des Deflektionspunkts, des Wendepunkts und/oder des Minimums.

10. Verfahren gemäss Anspruch 9, wobei ein PEEP-Wert auf Grundlage des charakteristische exspiratorischen Kurvenpunkts festgelegt wird.

11. Verfahren gemäss Anspruch 10, wobei der PEEP-Wert als Steuergrösse an das Beatmungsgerät ausgegeben.

12. Verfahren gemäss Anspruch 11 oder 12, wobei die Beatmung auf Grundlage des PEEP-Werts durchgeführt wird.

13. Verfahren gemäss einem der Ansprüche 9-12, wobei das Verfahren vor einer Beatmung und/der während der Beatmung, insbesondere turnusgemäss, durchgeführt wird.

14. Computerprogramm mit Programmcode zur Durchführung der Schritte des Verfahrens gemäss einem der Ansprüche 9 bis 13, wenn das Programm auf einem Computer ausgeführt wird.

15. Computerprogrammprodukt, das direkt in einen internen Speicher eines Digitalcomputers geladen werden kann und das Softwarecodeabschnitte umfasst, die die Verfahrensschritte von mindestens einem der Ansprüche 9 bis 13 ausführen, wenn das Programm auf dem Digitalcomputer läuft.
